# EUROPÄISCHE PATENTSCHRIFT

(11) **EP 0 554 422 B1**
(45) Veröffentlichungstag und Bekanntmachung des Hinweises auf die Patenterteilung: **30.08.1995**
(21) Anmeldenummer: 92916898.7
(22) Anmeldetag: 05.08.1992
(51) Int. Cl.: A61K 7/06

(54) **VERWENDUNG VON DL-VALIN, DESSEN ESTERN UND/ODER SALZEN ZUR BEKÄMPFUNG VON KOPFSCHUPPEN**
USE OF DL-VALIN, ITS ESTERS AND/OR ITS SALTS IN THE TREATMENT OF DANDRUFF
UTILISATION DE LA DL-VALINE, DE SES ESTERS ET/OU DE SES SELS POUR LUTTER CONTRE LES PELLICULES

(30) Priorität: 28.08.1991 DE 4128518
(43) Veröffentlichungstag der Anmeldung: 11.08.1993
(73) Patentinhaber: Wella Aktiengesellschaft, 64295 Darmstadt (DE)
(72) Erfinder: RÖTHLISBERGER, Rudi, CH-1723 Marly (CH); SCHNETZ, Michel, CH-1723 Marly (CH); KAUFMANN, Walter, D-6109 Mühltal 3 (DE)
(86) Internationale Anmeldenummer: EP9201769
(87) Internationale Veröffentlichungsnummer: WO9304661

(56) Entgegenhaltungen:
- EP-A- 0 413 528
- DE-A- 3 541 485

## Beschreibung

Gegenstand der vorliegenden Erfindung ist die Verwendung von DL-Valin, dessen Estern und/oder Salzen zur Bekämpfung von Kopfschuppen.

Es sind bereits zahlreiche Substanzen als Wirkstoffe zur Bekämpfung der Kopfschuppen ("Antischuppenwirkstoffe") empfohlen worden. Hierzu gehören Verbindungen wie kolloidaler Schwefel, Hydroxychinoline, Phenole, quartäre Ammoniumverbindungen, Selendisulfid, Pyridinthione, Thiazolverbindungen und zahlreiche andere Verbindungen.

Von den genannten Verbindungen weisen das 1-Hydroxy-2-pyridinthion und dessen Salze, insbesondere das Zinksalz, eine besonders gute Wirksamkeit bei Kopfschuppen auf. Aufgrund der in üblichen Lösungsmitteln wie Wasser und Alkoholen begrenzten Löslichkeit des Zink-Pyridinthions kann es jedoch nur unter Schwierigkeiten in klare kosmetische Mittel eingearbeitet werden.

Die bisher als Wirkstoffe zur Behandlung der Kopfschuppen vorgeschlagenen Verbindungen können hinsichtlich ihrer Wirksamkeit gegen Kopfschuppen in toxikologischer und dermatologischer Hinsicht, oder - wie im Falle des Zink-Pyridinthions - wegen der Schwerlöslichkeit in kosmetischen Lösungsmitteln, den gestellten Anforderungen nicht völlig zufriedenstellend genügen.

Zur Lösung dieses Problems wurde in der eigenen DE-OS 29 37 184, DE-OS 29 38 418, DE-OS 30 45 340 und DE-OS 38 03 783 vorgeschlagen, bestimmte 1,3-Thiazolidine oder Tetrahydro-1,3-thiazinderivate als Antischuppen-Wirkstoffe einzusetzen. Die Verbindungen weisen zwar eine bessere Löslichkeit in den für kosmetische Mittel gebräuchlichen Lösungsmitteln auf, jedoch ist ihre Antischuppenwirkung nicht völlig zufriedenstellend.

Alle bis heute bekannten Antischuppen-Wirkstoffe weisen zudem einen kosmetischen Nebeneffekt auf, der von der Mehrzahl der Verwender nicht akzeptiert werden kann: Eine starke Haarbelastung im Sinne einer stärkeren Nachfettung der Kopfhaut sowie des Haares. Die Ursache für diese Nebenwirkung ist bis heute ungeklärt. Es wurden zahlreiche Hypothesen aufgestellt, die nach experimenteller Überprüfung jedoch nicht bestätigt werden konnten. Untersuchungen an breiten Probandenkollektiven zeigten, daß keine Stimulierung der Sebumproduktion durch die Antischuppen-Wirkstoffe erfolgt; eine wesentliche Veränderung der Sebum-Zusammensetzung konnte ebenfalls nicht nachgewiesen werden. Unabhängig auch von anderen Hypothesen, die aufgestellt worden sind, stellt diese Haarbelastung für die betroffenen Verwender ein kosmetisches Problem dar.

Weiterhin handelt es sich bei den heute am häufigsten verwendeten gegen Kopfschuppen wirksamen Verbindungen, wie zum Beispiel Selendisulfid, Zinkpyridinthion oder 1-(4-Chlorphenoxy)-1-(1-imidazoyl)-3,3-dimethyl-2-butanon ("Climabazol"), um Substanzen, welche zwar eine gute Wirksamkeit gegen Kopfschuppen besitzen, deren physiologische Verträglichkeit jedoch nicht unumstritten ist.

Es bestand daher die Aufgabe, einen Wirkstoff gegen Kopfschuppen zur Verfügung zu stellen, der die vorstehend genannten Nachteile, insbesondere bezüglich der Löslichkeit, der physiologischen Verträglichkeit sowie der Nachfettung der Haare, nicht aufweist.

Es wurde nun überraschenderweise gefunden, daß DL-Valin, DL-Valinester oder DL-Valinsalze,alleine oder in Kombination miteinander, diese Aufgabe in hervorragender Weise lösen.

Insbesondere weisen DL-Valin, DL-Valinester und DL-Valinsalze eine gute Wirksamkeit gegen Kopfschuppen und eine ausgezeichnete physiologische Verträglichkeit auf und lassen sich aufgrund ihrer guten Löslichkeit in den üblicherweise in kosmetischen Mitteln verwendeten Lösungsmitteln problemlos in klare kosmetische Zubereitungen einarbeiten.

Im Gegensatz zu den üblicherweise verwendeten Antischuppenwirkstoffen tritt bei Verwendung von DL-Valin, dessen Salzen oder Estern keinerlei Nachfettung der Haare auf.

Gegenstand der vorliegenden Erfindung ist daher die Verwendung von DL-Valin und/oder DL-Valinestern und/oder DL-Valinsalzen zur Bekämpfung von Kopfschuppen.

Als DL-Valinester kommen vorzugsweise DL-Valinmethylester und DL-Valinethylester in Betracht, während als DL-Valinsalze die physiologisch verträglichen Salze des DL-Valins, insbesondere DL-Valinhydrochlorid, DL-Valinsulfat und DL-Valinacetat, zu nennen sind.

Gemäß der vorliegenden Erfindung sollen DL-Valin, DL-Valinester und/oder DL-Valinsalze in beliebigen, für die Haar- und Kopfbehandlung geeigneten kosmetischen Zubereitungen auf wäßriger, alkoholischer oder wäßrig-alkoholischer Basis, wie zum Beispiel in Einlegemitteln, Haarspülungen, Frisiergels, Frisiercremes, Haarkuren, Haarfestigern oder Haarsprays, vorzugsweise jedoch in Shampoos und Haarwässern, in einer Menge von 0,05 bis 10 Gewichtsprozent Verwendung finden.

Es handelt sich dabei um Zubereitungen, die je nach ihrem Anwendungszweck für kürzere oder längere Zeit auf dem Haar und der Kopfhaut verbleiben. Durch ihren Gehalt an DL-Valin und/oder DL-Valinestern und/oder DL-Valinsalzen wird hierbei gleichzeitig eine Schuppenbehandlung bewirkt. Es ist jedoch auch möglich, Zubereitungen herzustellen, die hauptsächlich oder ausschließlich dem Ziel einer Schuppenbekämpfung dienen.

Die Menge an DL-Valin und/oder DL-Valinestern und/oder DL-Valinsalzen beträgt in den Mitteln zur Behandlung der Haare und der Kopfhaut, welche nach der Anwendung nicht ausgespült werden und daher auf dem Haar und der Kopfhaut verbleiben, wie zum Beispiel in Haarwässern und Einlegemitteln, etwa 0,05 bis 1 Gewichtsprozent vorzugsweise 0,1 bis 0,5 Gewichtsprozent. Mittel, die kurz nach ihrer Anwendung ausgespült werden, wie beispielsweise Shampoos und Haarspülungen,enthalten das DL-Valin,die DL-Valinester und/oder die DL-Valinsalze in einer Menge von etwa 0,5 bis 10 Gewichtsprozent, vorzugsweise in einer Menge von 2 bis 5 Gewichtsprozent. Hierbei können die genannten Verbindungen jeweils allein oder im Gemisch miteinander in diesen Zubereitungen vorliegen.

Der pH-Wert dieser kosmetischen Zubereitungen beträgt 3 bis 9, vorzugsweise 5 bis 8.

Die Zusammensetzung dieser kosmetischen Zubereitungen stellt eine Mischung von DL-Valin und/oder DL-Valinestern und/oder DL-Valinsalzen mit den für solche Zubereitungen üblichen Bestandteilen, wie zum Beispiel Träger- und Zusatzstoffen, dar.

Der kosmetische Trägerstoff kann ein für die örtliche Anwendung üblicher Trägerstoff, wie eine Salbengrundlage oder vor allem ein flüssiger Trägerstoff, wie Wasser, Alkohole oder wäßrig-alkoholische Mischungen, sein. Hierfür geeignete Alkohle sind beispielsweise Ethanol, n-Propanol, i-Propanol sowie auch mehrwertige Alkohole wie Glycerin und Propylenglykol.

Flüssige Trägerstoffe wie Wasser und Alkohole sind deshalb besonders bevorzugt, weil sich bei ihrer Verwendung meist klare Lösungen ergeben und diese Trägerstoffe eine besonders intensive Benetzung der Kopfhaut ermöglichen.

Als übliche Zusatzstoffe in den kosmetischen Zubereitungen kommen beispielsweise kosmetische Harze; Emulgatoren; anionische, kationische, nicht-ionische oder amphotere Tenside; Verdicker, wie zum Beispiel höhere Fettalkohole, Stärke; Cellulosederivate, Paraffinöl, ferner Pflegestoffe, wie beispielsweise Lanolinderivate, Cholesterin oder Pantothensäure; sowie weiterhin Farbstoffe; Parfümöle; Treibgase und andere in Betracht.

Die nachstehenden Beispiele sollen den Gegenstand der Erfindung näher erläutern ohne ihn hierauf zu beschränken.

### Beispiele

### Beispiel 1 Klares Haarshampoo

5,0 g DL-Valin 11,2 g Natriumlaurylalkoholdiglykolethersulfat
4,0 g Natriumchlorid
0,2 g Parfümöl
79,6 g Wasser
100,0 g

### Beispiel 2 Klares Haarwasser

0 5 g DL-Valin
0,2 g Parfümöl
50,0 g Isopropanol
49,3 g Wasser
100,0 g

### Beispiel 3 Einlegemittel

0,5 g DL-Valinmethylester
3,0 g Vinylpyrrolidon/Vinylacetat-Copolymerisat 60/40 (Luviskol@ VA 64 der Firma BASF, Ludwigshafen/BRD)
0,2 g Parfümöl
40,0 g Ethanol
56,3 g Wasser
100,0 g

## Patentansprüche

1. Kosmetische Verwendung von DL-Valin und/oder DL-Valinestern und/oder DL-Valinsalzen zur Bekämpfung von Kopfschuppen.

2. Verwendung von DL-Valin und/oder DL-Valinestern und/oder DL-Valinsalzen nach Anspruch 1 in einer Menge von 0,05 bis 10 Gewichtsprozent in einer kosmetischen Zubereitung auf wäßriger, alkoholischer oder wäßrig-alkoholischer Basis.

3. Verwendung von DL-Valin und/oder DL-Valinestern und/oder DL-Valinsalzen nach Anspruch 1 oder 2 in einer Menge von 0,05 bis 1 Gewichtsprozent in Mitteln zur Behandlung der Haare und der Kopfhaut, welche nach der Anwendung nicht ausgespült werden.

4. Verwendung von DL-Valin und/oder DL-Valinestern und/oder DL-Valinsalzen nach Anspruch 1 oder 2 in einer Menge von 0,5 bis 10 Gewichtsprozent in Mitteln zur Behandlung der Haare und der Kopfhaut, welche nach der Anwendung ausgespült werden.

5. Verwendung nach einem der Ansprüche 1 bis 4, dadurch gekennzeichnet, daß der DL-Valinester ausgewählt ist aus DL-Valinmethylester und DL-Valinethylester.

6. Verwendung nach einem der Ansprüche 1 bis 4, dadurch gekennzeichnet, daß das DL-Valinsalz ausgewählt ist aus DL-Valinhydrochlorid, DL-Valinsulfat und DL-Valinacetat.

7. Verwendung nach einem der Ansprüche 1 bis 6, dadurch gekennzeichnet, daß das DL-Valin und/oder der DL-Valinester und/oder das DL-Valinsalz in einer kosmetischen Zubereitung zur Anwendung gelangt, die als Haarspülung, Einlegemittel, Frisiergel, Frisiercreme, Haarkur, Haarfestiger, Haarspray, Shampoo oder Haarwasser vorliegt.

## Claims

1. Cosmetic use of DL-valine and/or DL-valine esters and/or DL-valine salts for the treatment of dandruff.

2. Use of DL-valine and/or DL-valine esters and/or DL-valine salts according to Claim 1 in a quantity of from 0.05 to 10 weight % in a water- based, alcohol-based or water-alcohol-based cosmetic preparation.

3. Use of DL-valine and/or DL-valine esters and/or DL-valine salts according to Claim 1 or Claim 2 in a quantity of from 0.05 to 1 weight %, in hair and scalp treatment preparations which are not rinsed out after use.

4. Use of DL-valine and/or DL-valine esters and/or DL-valine salts according to Claim 1 or Claim 2 in a quantity of from 0.5 to 10 weight % in hair and scalp treatment preparations which are rinsed out after use.

5. Use according to any one of Claims 1 to 4, characterised in that the DL-valine ester is selected from DL-valine methyl ester and DL-valine ethyl ester.

6. Use according to any one of Claims 1 to 4, characterised in that the DL-valine salt is selected from DL-valine hydrochloride, DL-valine sulphate and DL-valine acetate.

7. Use according to any one of Claims 1 to 6, characterised in that the DL-valine and/or the DL-valine ester and/or the DL-valine salt is presented for use in cosmetic preparation available as a hair rinse, a hair-setting preparation, a hair gel, a hair cream, a hair treatment, a hair-setting lotion, a hair spray, a shampoo or a hair lotion.

## Revendications

1. Utilisation cosmétique de DL-valine et/ou d'esters de DL-valine et/ou de sels de DL-valine pour la lutte contre les pellicules

2. Utilisation de DL-valine et/ou d'esters de DL-valine et/ou de sels de DL-valine selon la revendication 1 à raison de 0,05 à 10% en poids dons une préparation cosmétique à base aqueuse, alcoolique ou hydro-alcoolique.

3. Utilisation de DL-valine et/ou d'esters de DL-valine et/ou de sels de DL-valine selon la revendication 1 ou 2, à raison de 0.05 à 1 % en poids dans un milieu de traitement des cheveux et du cuir chevelu qui ne sont pas rincés après utilisation.

4. Utilisation de DL-valine et/ou d'esters de DL-valine et/ou de seis de DL-valine seton la revendication 1 ou 2 à raison de 0.5 à 10% en poids dans des milieux de traitement des cheveux et du cuir chevelu qui sont rincés après utilisation.

5. Utilisation selon l'une des revendications 1 à 4, caractérisée en ce que 1 'ester de DL-valine est choisi parmi l'ester de méthyle de DL-valine et l'ester d'éthyle de DL-valine.

6. Utilisation selon l'une des revendications 1 à 4, caractérisée en ce que le sel de DL-valine est choisi parmi le chlorhydrate de DL-valine, le sulfate de DL-valine, et 1 'acétate de DL-valine.

7. Utilisation selon 1 'une des revendications 1 à 6, caarctérisée en ce que DL-valine et/ou d'esters de DL-valine et/ou de sels de DL-valine est présente dans une préparation cosmétique pour utilisation qui se présente sous forme de produit de rinçage des cheveux, agent de mise en pli , gel de coiffage, crème de coiffage, produit de soins des cheveux, fixateur des cheveux, porduits pulvérisés pour cheveux, shampooing ou lotion capillaire.
